Europäisches Patentamt

European Patent Office

Office européen des brevets

(19)

(11) Publication number: 0 349 769

A1

## (12) EUROPEAN PATENT APPLICATION

(21) Application number: 89110034.9

(22) Date of filing: 02.06.89

(51) Int. Cl.4: C12N 15/00 , A01N 63/02 , C12N 1/20 , //(C12P1/04, C12R1:07)

The microorganism(s) has (have) been deposited with Collection Nationale de Cultures de Mikroorgasmes under number(s) CNCM I-746.

(30) Priority: 05.06.88 IL 86623

(43) Date of publication of application:
10.01.90 Bulletin 90/02

(84) Designated Contracting States:
AT BE CH DE ES FR GB GR IT LI LU NL SE

(71) Applicant: BIOTECHNOLOGICAL
APPLICATIONS LTD.
P.O. Box 4309
Jerusalem 91042(IL)

(72) Inventor: Sandler, Naomi
29 Kafar Atzion
Jerusalem 93392(IL)
Inventor: Bar, Elvira
5/30 Hahagana Street Hagiva Hatzarfatit
Jerusalem 97852(IL)
Inventor: Bashkin, Phina
339/4 Ramot "G"
Jerusalem 97725(IL)
Inventor: Hurwitz, Judith
555/15,Giloh
Jerusalem(IL)
Inventor: Keynan, Alex
16 Balfur Street
Jerusalem 92102(IL)

(74) Representative: Patentanwälte Grünecker,
Kinkeldey, Stockmair & Partner
Maximilianstrasse 58
D-8000 München 22(DE)

(54) Genetically engineered bacillus sphaerocus producing larvicidal toxin from bacillus thuringiensis var. israelensis.

(57) Bacillus sphaericus (B. sph.) are transformed with the toxin encoding gene from Bacillus thuringiensis var. israelensis (B.t.i.) and consequently new strains of B. sph. are produced having both the wide spectrum activity against larvae of mosquities and black flies of (B.t.i.) and the natural persistence characteristic of B. sph.

The newly produced B. sph. strains are useful as bio-insecticides and as such are applied to water reservoirs in which larvae of mosquitos and black flies live.

# Genetically engineered Bacillus sphaericus producing larvicidal toxin from Bacillus thuringiensis var. israelensis

## FIELD OF INVENTION

The present invention concerns new strains of Bacillus sphaericus which have been genetically engineered to produce the larvicidal toxin of Bacillus thuringiensis var. israelensis (B.t.i.), and their use as insecticides.

## BACKGROUND OF INVENTION AND PRIOR ART

Due to the hazardous consequences of conventional pesticides, there is a growing interest in using bio-pesticides, i.e. biological organisms (usually micro-organisms) which have the capability of controlling the pest.

Among the most common pests are mosquitoes and black flies (Simulium), many of which are vectors of various diseases, especially tropical ones. Mosquitos of the genera Anopheles, are carriers of malaria which affects many millions of people throughout the world. Mosquitoes of the genus Culex are carriers of viral encephalitis and, in tropical and sub-tropical climates, of philariasis. Mosquitoes of the genus Aedes . are carriers of yellow fever, dengue, and encephalitis.

Some species of black flies carry worms which are capable of causing onchocerciasis, which may result in blindness or in nodules beneath the skin. In spring, along the Mississippi River, for example, a certain species of black fly, Cnephia pecuarum, is a serious livestock pest and there are records of this species killing horses and mules either with blood sucking bites or by smothering, which may occur when the animals nostrils become blocked by large numbers of the insect.

The larvae and pupae of both mosquitos and black flies live in water. Thus, the easiest way to control mosquitos and black flies is by killing the larvae in the water reservoirs in which they develop. However, it is clear that the addition of insecticides into the water greatly affects its quality and has hazardous environmental consequences. Bacillus thuringiensis israelensis (B.t.i.) is a bacterium which produces a highly effective and specific mosquito larval toxin during the sporulation segment of its life cycle. Larvae of all the major disease-bearing mosquito species and also of Simulium (black flies) are affected (Goldberg and Margalit, 1977). The toxin appears in the form of an amorphous parasporal crystal which is made up of several major proteins of approximate molecular sizes 28kDa, 65kDa, 128kDa and 135kDa (Huber et al., 1981; Tyrell et al., 1981; Armstrong et al., 1985). The crystals are solubilized at the alkaline pH of the midgut of susceptible insect larvae and release the active toxin, whose effects include hemolysis of mammalian red blood cells and destruction of larval gut epithelial cells. The presence of the 28kDa protein has often been associated with both hemolysis and with toxicity to larvae (Thomas and Ellar, 1983; Yamamoto et al., 1983; Davidson and Yamamoto, 1984; Armstrong et al., 1985), although toxicity has also been attributed to the other crystal proteins, either by themselves or in combination (Hurley et al., 1985; Wu and Chang, 1985; Chilcott and Ellar, 1988; Delecluse et al., 1988).

The major limitation in using B.t.i. as an insecticide is its short effective lifetime in water containing inorganic particulate matter or decomposing organic material (Van Essen and Hembree, 1982; Ramoska et al., 1982; Margalit and Bobroglo, 1984). The rapid disappearance of the larvicidal activity of B.t.i. from the surface of bodies of water, where the larvae of most mosquito and black fly species feed, and to where the larvae must come for fresh air, make it economically impractical to use B.t.i., especially in the tropical areas, this being the major reason for its limited use today. Bacillus sphaericus (B. sph.) is another spore-forming bacterium which produces a toxin (having a molecular weight of about 110kDa) which kills mosquito larvae during sporulation. Although this toxin is highly effective against susceptible larvae, the target spectrum of B. sph. is more limited than that of B.t.i. and does not include either Aedes mosquitos or Simulium. However, B. sph. exerts a much longer effective control in mosquito breeding sites than B.t.i., possibly due to an ability to multiply and sporulate in the mosquito larvae or in the natural environment.

Thus, a combination of the broad target spectrum of B.t.i. with the natural long-term effect of B. sph. would have been greatly desirable and is the object of the present invention.

## SUMMARY OF THE INVENTION

In accordance with the invention, a DNA containing the toxin gene from B.t.i. was inserted into plasmids which were then used to transform B. sph. cells. The transformation resulted in new strains of B. sph. expressing both the B.t.i. toxin protein and the B. sph.'s own toxin.

In accordance with the invention, there is thus provided a genetically engineered B. sph. transformed with a DNA sequence from B.t.i. encoding the B.t.i. insect larval toxin protein (hereinafter "B.t.i. toxin").

The present invention also provides new plasmids containing a DNA sequence from B.t.i. encoding the B.t.i. toxin.

The genetically engineered B. sph. strains in accordance with the invention, to be referred to hereinafter at times as "B. sph. transformants", express the 110kDa B. sph. endotoxin protein and are also capable of expressing the 28kDa, 65kDa, 128kDa and/or the 135 kDa toxin proteins from B.t.i., of which the 128kDa and 135kDa are the proteins and the 28kDa and the 65kDa are the toxins themselves. Thus, the new transformants can display both the broad spectrum larvicidal activity of B.t.i. and the specific activity of B. sph., they have the long persistence characteristic of the latter and are thus very useful in combatting mosquitoes and black flies.

The toxins are released by the bacterial cells during sporulation and the mixture of the toxins and the spores may then be formulated into larvicidal compositions.

The present invention thus provides compositions for destroying larvae of mosquitos and black flies comprising an effective amount of B.t.i. toxin produced by the B. sph. transformants or spores of B. sph. transformants, or a combination of the two, and a carrier compatible with both the spores and the water environment.

Furthermore, the invention also provides methods for controlling mosquitos or black flies comprising applying into water reservoirs or running water in which the insects' larvae grow, an effective amount of B.t.i. toxin produces by the B. sph. transformants or B. sph. transformants or a combination of the two, together with a carrier compatible with both the bacterial spores and the water environment.

## DETAILED DESCRIPTION OF THE INVENTION

The gene encoding the B.t.i. toxin (hereinafter the "B.t.i. toxin gene") is known to be carried on a plasmid which has 112 kilobases (hereinafter "112 kb plasmid") and has a molecular weight of about 72-75 megadaltons (Ward and Ellar, 1988; Gonzales and Carlton, 1984). As mentioned above, the encoded toxin can be dissociated into several components, of which the 28 kDa is probably responsible for both the larvicidal activity and the hemolysis of erythrocytes by alkali-treated toxin.

The enzyme Hind III may advantageously be used in order to isolate the B.t.i. toxin gene from the 112 kb plasmid since there are no restriction sites for that enzyme within the gene itself.

A suitable cloning vehicle for the gene is the plasmid pPL603E, which is a derivative of the pUB110 which codes for resistance to neomycin or kanamycin and chloramphenicol (Duvall et al., 1984; 1987). This plasmid contains a single Hind III site within the chloramphenicol acetyl-transferase (cat) gene and it includes a promoter which, when the plasmid is inserted into B. subtilis, is activated during both vegetative growth and sporulation, and also contains a segment conferring inducibility by Cm (Mongkolsuk et al., 1984; Duvall et al., 1984).

It should be noted that the present invention is not limited to the use of pPL603E as the cloning vehicle, nor to the use of Hind III in cutting the plasmid and preparing segments of the gene for cloning. On the contrary, the present invention can also be carried out with other plasmids known in the art, and other enzymes may be used in order to cut the plasmid and the gene.

The invention will hereinafter be described with reference to the use of pPL603E plasmid as the cloning vehicle and the use of the Hind III enzyme, it being understood that this does not constitute a limitation of the invention.

After cutting both the plasmid and the gene with Hind III, a ligation mixture is formed which comprises the Hind II cut gene, the Hind III-cut plasmid and a DNA ligase such as, for example, T4 DNA ligase.

Bacterial cells may then be transformed by methods known in the art such as, for example, the method of Chang and Cohen (1979) or the method by McDonald and Burke (1984). Optimal results in transformation were found by a modification of the method of McDonald and Burke originally devised for B. sph. of the strain 1593. Briefly, in the modified method, the cells are grown in MBS medium (Kalfron et al., 1983) until early exponential phase, then lysozyme is added and upon formation of protoplast, polyethylene glycol is added and as a result, plasmid DNA enters the bacterial protoplast. The protoplasts are then grown in a suitable medium for the regeneration of the cell wall.

The transformed B. sph. cells can then be transferred into a selection medium. Such a medium will comprise, in addition to nutrients, also kanamycin or neomycin and consequently only the bacteria containing a plasmid which confers resistance to kanamycin and neomycin will grow and form colonies. The Hind III restriction site of the plasmid pPL603E is within the chloramphenicol resistance gene, and thus bacterial cells containing plasmids which do not have a DNA sequence inserted within that gene will retain both chloramphenicol and kanamycin (and neomycin) resistance, while bacterial cells containing plasmids having a DNA sequence inserted therein will lose the chloramphenicol resistance property but retain the neomycin and kanamycin resistance.

Accordingly, a sample of the transformants B. sph. from colonies which grew on a kanamycin or neomycin substrate, will be transferred to a chloramphenicol-containing substrate. Bacterial growth on the latter substrate will indicate that no foreign DNA has been inserted into the plasmid, while no-growth indicates the contrary.

DNA hybridization tests, e.g. the method of Maniatis et al. (1982), may be employed in order to check whether the foreign DNA sequence in the transforming plasmid is the B.t.i. toxin gene.

In order to test if the transformed bacterial cells secrete the B.t.i. toxin, various immunoassays may be employed. Furthermore, the toxicity of the B. sph. transformant on insect larvae may be tested by various bio-assays known in the art.

Once a B. sph. transformant clone has been obtained, it may be maintained as a pure continuous culture or may be lyophilyzed or frozen for long periods of time.

For the preparation of a larvicidal composition, large quantities of the B. sph. transformant are required, which are obtained by fermentation. B. sph. generally does not metabolize sugars and requires amino acids and vitamins and is thus grown in media containing a variety of proteinaceous substances. A suitable medium for growing B. sph. is one which comprises various inexpensive protein hydrolysates supplemented with a non-carbohydrate carbon source and an alternative nitrogen source.

The B. sph. transformants are grown in the fermentation medium until the termination of the sporulation and the beginning of the lysis of the sporulated bacterial cells upon which the toxin protein crystals are released.

Larvacidal compositions of transformed B. sph. may either be dry or liquid compositions. A dry composition may comprise toxin crystals and lyophilyzed or spray-dried spores of the transformed B. sph., possibly with various additives compatible with the spores and the water environment. A liquid composition may be a suspension of the spores together with the medium in which they were grown, which will thus comprise also toxin protein crystals. A suitable concentration of the transformed B. sph. bacteria in a liquid formulation is about $10^{10}$ spores.

## DESCRIPTION OF SPECIFIC EMBODIMENTS

The invention will now be described with reference to some specific examples which should not be construed as limiting the present invention, but rather as an illustration. The man of the art will be able, using the teaching of the present invention, to carry out the invention in its full scope as defined in the claims.

Statement of Deposit

A B. sph. transformant clone which was termed Sph. T4, containing a modified pPL603E which includes the 9.7 Kb Hind III segment of the B.t.i. toxin gene, was deposited in the Institut Pasteur, Collection Nationale de Cultures de Microorganismes (CNCM), the details of the deposition being as follows:
Deposition date : April 5, 1988
Accession number: CNCM I-746

Example 1: Media

The following media were used in the following examples:

a) Media for growth

| MBS (A. Kalfon et al., 1983) | | | | | |
|---|---|---|---|---|---|
| KH$_2$PO$_4$ | - | 50 mM | ZnSO$_4$ 7H$_2$O | - | 0.07 mM |
| MgSO$_4$ | - | 7H$_2$O 12 mM | Fe$_2$ (SO$_4$)$_3$ | - | 5 mM |
| MgSO$_4$ | - | 0.01 mM | CaCl$_2$ | - | 1 mM |
| Tryptose (Difco) 10 g/l | | | | | |
| Yeast extract 2 g/l | | | | | |
| pH = 7.2 | | | | | |

<u>MS</u>

For 1 liter 16 gm nutrient broth (purchased from DIFCO, Detroit, U.S.A.), 2 gm KCl (plus 15 gm Bacto agar for plates). After autoclaving, the following salts and glucose were added to give a final concentration of: 0.05% MgSO$_4$, 7H$_2$O, 1 mM Ca(NO$_3$)$_2$, 0.1 mM MnCl$_2$, 1$\mu$M Fe SO$_4$, and 0.5% glucose.

b) Medium for regeneration

| DM-3 | | | | | |
|---|---|---|---|---|---|
| Agar | - | 1.1% | KH$_2$PO$_4$ | - | 0.15% |
| Sodium succinate | - | 0.5 M | Glucose | | 0.5% |
| Casamino acids | - | 0.5% | MgCl$_2$ | - | 0.02 M |
| Yeast extract | - | 0.5% | BSA | - | 0.01% |
| K$_2$HPO$_4$ | - | 0.35% | | | |

c) Media for transformation

<u>MM2</u>

Penassay broth x 2
Yeast extract 0.5%

<u>SMMP</u>

Equal volumes of:
i) Penassay broth x 4
ii) SMM Buffer x 2
BSA 1%

d) Solutions and buffers

<u>TE Buffer</u>

Tris HCl 10 mM
EDTA mM
pH = 8.0

<u>SMM Buffer</u>

Sucrose 0.5 M
Maleate 0.02 M

MgCl$_2$ 0.02 M
pH = 6.5

PEG Solution

40 g PEG (Sigma m.w. 6000)
50 ml 2 x SMM buffer
Adjust to 100 ml with H$_2$O

Example 2: Preparation of Recombinant DNA and Transformation of B. Sph.

B.t.i. DNA was prepared by a method which yields preferentially the DNA of large plasmids (Casse et al., 1979) and both vector (pPL603E) and donor DNA were digested with Hind III and ligated with T4 DNA ligase.

Transformation of B. sphaericus was generally performed by the prostoplast transformation method originally developed by Chang and Cohen (1979) and adapted to B. sph. 1593 by McDonald and Burke (1984), which was, however, modified in order to yield optimum results with strain 2362.

For transformation, a colony of B. sph. 2362 was inoculated and grown in MM2 medium at 37° with shaking until early exponential phase was reached. The culture was then harvested by centrifugation for 10 minutes at 1600 RPM and the pellet was resuspended in 1/6 volume of SMMP medium. Lysozyme (600 µg/ml) was added and the cells were incubated at 37° with gentle shaking for one hour.

Cells were examined under the microscope for protoplast formation and then pelleted by centrifugation at 1400 RPM for 15 minutes at room temperature. The pellet was suspended in a/15 of the original culture volume in SMMP.

The ligated DNA was suspended in 50 µl TE buffer and then an equal volume of 2 x SMM was added. 0.5 ml protoplast solution plus 1.5 ml 40% PEG/SMM buffer were added and the mixture was vortexed gently for 2 minutes. Thereafter, 5 ml of SMMP medium was added and the bacterial cell suspension was centrifugated twice for 10 minutes at 1400 RPM at room temperature. The pellet was resuspended in 1 ml SMMP and incubated for 2 hours at 30° C.

After transformation, the bacterial cells were transferred into the DM-3 medium for regeneration.

Following the regeneration of the cell walls, the bacterial cells were tested for the transformed phenotype. Transformed clones containing B.t.i. DNA were identified by the Kanamycin (Kn) resistance derived from the presence of plasmid pPL603E, together with sensitivity to Chloramphenicol (Cm) due to the insertion of foreign DNA at the Hind III site within the cat gene of the plasmid. 500 colonies from plates of protoplast regeneration medium were transferred in parallel to plates of nutrient agar containing either Kn (10 µg/ml) or Cm (50 µg/ml). Kn resistant, Cm sensitive clones constituted approximately 60% of the total colonies. About 8% of the transformants containing recombinant DNA were found to be very stable, in that they did not lose the transformed phenotype after 20 passages of cultures in the absence of Kn. An additional 20% were moderately stable, and could be transferred 5 times before losing Kn resistance. The remaining approximately 72% lost Kn resistance immediately on being grown on plates not containing Kn.

One of the transformed B. sph. which was obtained was CNCM I-746.

Example 3: Method for identification of colonies transformed with B.t.i. toxin gene

a) DNA hybridization

The hybridization was performed according to the method of Maniatis et al. (1982). Briefly, the 9.7 Kb B.t.i. toxin gene (obtained from digestion with Hind III) was radioactively labelled with P$^{32}$ by the method of nick-translation, and the so-labelled DNA was then incubated with DNA taken from the clone to be tested.

b) Immunoassay

The lyophilized material was resuspended at 2 mg/ml in sodium carbonate (pH = 10.5) and placed at 37° C for 30 min. 2µl of each sample were spotted on nitrocellulose filters and the filters were placed in petri dishes and incubated in 5 ml of pre-incubation buffer containing 5% low fat milk powder in 50 mM Tris-HCl (pH = 7.5) and 150 mM NaCl for two hours at 37° C on a shaker. The preincubation buffer was discarded. Antibody against the 28 or 65kDa crystal protein of B.t.i. or against 110 kd crystal protein of B. sphaericus were diluted 1:500 in pre-incubation buffer and pipetted onto the filter. The filter was then left at room temperature overnight.

Each filter was washed in 0.2% triton X-100, 100 mM Tris-HCl (pH 7.5), 100 mM NaCl, 5 mM MgCl₂ for 10 minutes at room temperature on a shaker. The filters were then washed 5 times in AP (pH = 7.5) buffer, (10 mM Tris-HCl pH = 7.5, 100 mM NaCl, 5 mM MgCl₂) on a shaker.

Each wash in AP buffer was for 15 minutes. The filters were then incubated for 2 hours on a shaker at room temperature with alkaline phosphatase linked to goat anti-rabbit IgG (from Bio Yedda) diluted at 1:1000 in A.P. (pH = 7.5) buffer. The filters were then washed twice in A.P. (pH = 7.5) buffer and four times in A.P. (pH = 9.5) buffer (100 mM Tris-HCl pH = 9.5, 100 mM NaCl, 5 mM MgCl₂). Each wash was for 10 minutes on a shaker at room temperature. For the colour reaction the following two solutions were prepared:
1) 1 mg nitro blue tetrazolium (NBT) in 3 ml A.P. pH = 9.5.
2) 0.5 mg 5-bromo-4-chloro-3 indolylphosphate (BCIP) in 15 μl of dimethyl formamide (DMD). The two solutions were vortexed together and poured over the filters. The filters were left at room temperature, with slight shaking, and the colour developed within several minutes. When the background began to darken the filters were rinsed twice in a 1.5 dilution of A.P. pH = 7.5. Then they were left to dry between 3 mm papers to protect them from light.

c) Toxin Bioassay

To test toxicity toward mosquito larvae, triplicate samples of 10 third instar larvae of either Culex pipiens or Aedes aegypti were placed in plastic cups containing 50 ml of water.

Suspensions of lyophilized powders of bacterial cultures were added at the desired concentration, and the cups were checked for the presence of dead larvae after standing at 27° C for 24 and 48 hours. The LD₅₀ (a dose which causes 50% mortality) was calculated as recommended by the WHO (Rishikesh and Quelennec, 1983).

Example 4: Identification of B.t.i. toxin DNA in stable transformed clones of B. sphaericus

DNA was isolated from four clones of the moderately stable and four clones of the highly stable groups of transformants, and hybridized with the 9.7 Kb Hind II fragment isolated from the 72mDa B.t.i. plasmid which carries the toxin genes, as described in Example 3a. This fragment reportedly codes for toxic activity (Ward et al., 1984), as was confirmed also by re-cloning this fragment into B. subtilis, producing clones which reacted antigenically with antibodies directed against the 28kDa crystal protein of B.t.i. and which also possessed a high larvicidal activity.

(Re-cloning of the 9.7 Kb into B. subtilis was performed in a similar manner, mutatis mutandis, as described in connection with the transformation of B. sph.)

The 9.7 Kb fragment did not hybridyze detectably with DNA from B. sph. 2362 or with DNA from the 4 moderately stable transformed clones which had been selected at random for this test. However, the 4 clones of the highly stable group contained DNA which hybridized with the fragment, thus indicating the presence of a corresponding gene in these transformed clones.

Example 5 Expression of B.t.i. toxin genes in the stable transformants of B. sphaericus

a) Immunoassay

Enzyme-linked immunoassay was performed as described in Example 3b). Cell extracts and culture supernatants from B.t.i. culture, from B. sph. 2362 before transformation, and from the transformed culture were exposed to antibodies to the 28 and 65kDa B.t.i. crystal protein toxin and to the 110 kDa B. sph. toxin protein. The results of the immunoassay are shown in the following Table I:

Table I

| Immunoassay of Transformed Clones* | | | | | | | | |
|---|---|---|---|---|---|---|---|---|
| | | | Pellet | | | Supernatant | | |
| | | | $\alpha$28 | $\alpha$65 | $\alpha$110 | $\alpha$28 | $\alpha$65 | $\alpha$110 |
| B.t.i. | | 12h | + /- | + | - | - | - | - |
| | | 24h | + + | + + | - | + /- | - | - |
| | | 48h | + + | + + | - | + /- | + /- | - |
| B. UND>sph. 2362 before transmation | | 12h | - | - | + | - | - | + /- |
| | | 24h | - | - | + + | - | - | + |
| | | 48h | - | - | + + | - | - | + |
| N.S. | | 12h | + /- | + /- | + | - | - | + |
| | | 24h | + + | + + | + + | + /- | + | + + |
| | | 48h | + | + | + + | + | + + | + + |
| *Legends used in Table | | | | | | | | |
| $\alpha$28 - Rabbit antibody against 28 kd crystal protein of B.t.i. | | | | | | | | |
| $\alpha$65 - Rabbit antibody against 65 kd crystal protein of B.t.i. | | | | | | | | |
| $\alpha$110 - Rabbit antibody against 110 kd crystal protein of B. sph. | | | | | | | | |
| N.S.- new strain | | | | | | | | |
| + - positive reaction | | | | | | | | |
| - - negative reaction | | | | | | | | |

The results of the above Table show that the new strain secretes both the B.t.i. and B. sph. 2362 toxin.

b) Bioassay

Suspensions of lyophilyzed powders prepared from B.t.i., B. sph. 2362 and transformed B. sph. 2362 cultures were assayed as described in Example 3c) for toxicity toward Culex pipiens, which is sensitive to both B.t.i. and B. sphaericus, and Aedes aegypti, which is sensitive only to B.t.i. The results are shown in the following Table II:

Table II

| Larvacidal activity of untransformed and transformed B. sphaericus | | | | | | | | |
|---|---|---|---|---|---|---|---|---|
| Sample Content | % Dead Aedes | | | | % Dead Culex | | | |
| | 24h | | 48h | | 24h | | 48h | |
| | c = 1 | c = 10 | c = 1 | c = 10 | c = 1 | c = 10 | c = 1 | c = 10 |
| | μg/ml | μg/ml | μg/ml | μg/ml | μg/ml | μg/ml | μg/ml | μg/ml |
| B.t.i. | 100 | 100 | 100 | 100 | 100 | 100 | 100 | 100 |
| B.t.i. + B. sph. 2362 | 100 | 100 | 100 | 100 | 100 | 100 | 100 | 100 |
| B. sph. 2362 | 0 | 10 | 15 | 20 | 75 | 65 | 100 | 100 |
| N.S. p-15 | 40 | 60 | 50 | 80 | 95 | 100 | 100 | 100 |
| N.S. p-17 | 100 | 100 | 100 | 100 | 100 | 100 | 100 | 100 |
| Legend: | | | | | | | | |
| B.t.i. = Bacillus thuriugiensis var. israelensis | | | | | | | | |
| B. sph. = Bacillus sphaericus | | | | | | | | |
| N.S. = New strain | | | | | | | | |
| P-# = number of transfers in selective conditions | | | | | | | | |
| c = final concentration of lypholized cells | | | | | | | | |

From the Table it may be concluded that the new strain of the present invention is toxic for larvae of both Culex and Aedes mosquitos. The larvicidal activity is stable in these strains through various transfers from plate to plate in selective conditions (p-15 and p-17 have undergone 15 and 17 transfers respectively).

The half-lethal dose concentration ($LC_{50}$ - in units of μg powder per ml) of various clones on the mortality of Aedes larva after 48 hours of exposure to powders prepared from sporulated cultures was compared with the intensity of the immunological response to the same powders, and the results are shown in the following Table III:

Table III

| Strain | IMMUNOLOGICAL RESPONSE | | | $LC_{50}$ μl |
|---|---|---|---|---|
| | B.t.i.-proteins | | B . sph . prot. | |
| | 28kDa | 65kDa | 110kDa | |
| B. sph. 2362 | - | - | + + + | 1.9 |
| Clones: | | | | |
| 62 | + + + | + | + + + | 0.19 |
| 5 | + | - | + + + | 0.57 |
| 13 | + + | - | + + + | 0.21 |
| 96 | - | + + | + + + | 1.7 |

The above results show that the transformed clone which expressed the 28kDA protein, as determined by the immunological assay, had an $LC_{50}$ which is about an order of magnitude less than that of B. sph. 2362. It should be noted, however, that the $LC_{50}$ of the transformed clone is about one order of magnitude higher than that of B.t.i.

Example 6: Fermentation and formulation

a) A medium for fermentation

| Minerals | Concentration (g/l) |
|---|---|
| $MgSO_4 \cdot 7H_2O$ | 0.3 |
| $MnSO_4$ | 0.02 |
| $Fe_2(SO_4)_3$ | 0.02 |
| $ZnSO_4 \cdot H_2O$ | 0.02 |
| $CaCl_2$ | 0.02 |
| Nitrogen Source: | Proteose peptone (DIFCO, U.S.A.) or Sheftones (Sheffield, U.S.A.) both at a concentration of 5-10 g/l |
| Carbon Source: | 5 g/l glycerol |

b) Fermentation of CNCM I-746

The fermentation medium described under a) above was mixed with 0.05% of antifoam (P-2000, Frutarum, Israel) and after inoculation with 0.1% inoculum of vegetative stage of the bacterial culture, fermentation proceeded as follows:

| | |
|---|---|
| Time: | 20-24 hours (terminated by view of sporulation and lysis) |
| Temperature: | 30° C |
| Aeration: | 0.8 VVM |
| Stirring: | 500 rpm |

The fermentation broth was harvested and the paste (15-20% dry weight) was collected. The paste was formulated as a liquid formulation by the addition of benzoic acid (2%) and Tween 80 (0.5%).

Freeze dried or spray dried powder of the paste was also prepared as a dry formulation.

References

1. Armstrong et al. (1985). J. Bacteriol. 161: 39-46.

2. Casse et al. (1979). J. Gen. Microbiol. 113: 229-242.

3. Chang and Cohen (1979). Molec. Gen. Genet. 168: 111-115.

4. Chilcott and Ellar (1988). J. Gen. Microbiol. 134: 2551-2258.

5. Davidson and Yamamoto (1984). Curr. Microbiol. 11: 171-174.

6. Delecluse et al. (1988). 10th International Spores Conference, March 23-27, Wood's Hole, Abstracts.

7., Dubnau et al. (1980). Genetic Engineering Vol. 2 Setlow and Hollander (eds).

8. Duvall et al. (1984). J. Bacteriol. 158: 784-790.

9. Duvall et al. (1987). J. Bacteriol. 169: 4235-4241.

10. Goldberg and Margalit (1977). Mosq. News. 37: 355-358.

11. Gonzalez and Carlton (1984). Plasmid. 11: 28-38.

12. Gryczan et al. (1978). J. Bacteriol. 134: 318-329.

13. Huber and Luthy (1981). Pathogenesis- of Invertebrate Microbiol Disease, pp. 206-216 (E.W.Davidson, ed.).

14. Hurley et al. (1985). Biochem. Biophys. Res. Commun. 126: 961-965.

15. Kalfon et al. (1983). Eur. J. Appl. Microbiol. Biotechnol. 18: 168-173.

16. Maniatis et al., eds. (1982). Cold Spring Harbor Laboratory.

17. Maniatis et al. (1984). J. Gen. Microbiol. 130: 203-208.

18. Margalit and Bobroglo (1984). Zeitschrift fur angewandte Entomologie 97: 516-520.

19. Mongkolsuk et al. (1984). J. Bacteriol. 160: 1-8.

20. Nyberg, K. (1985). FEMS Microbiol. Lett. 29 311-316.

21. Ramoska et al. (1982). J.Ecan. Entamol. 75: 1-4.

22. Rishikesh and Quennelec. (1983). Bulletin of the World Health Organization. 61: 93-97.

23. Sekar and Carlton. (1985). Gene. 33: 151-158.

24. Thomas and Ellar. (1983). J. Cell. Sci. 60: 181-197.

'25. Tyrell et al. (1981). J. Bacteriol. 145: 1052-1062.

26. Van Essen and Hembree (1982). Mosq. News 42: 1-66.

27. Ward et al. (1986). J. Mol. Biol. 191: 13-22.

28. Ward et al. (1984). FEBS Lett. 175: 377-381.

29. Ward and Ellar (1988) J. Bacteriol. 170: 727-735.

30. Wu and Chang (1985). FEBS Lett. 190: 232-236.

31. Yamamoto et al (1983). Curr. Microbiol. 9: 279-284.

32. Yousten, A.A. (1984). Adv. Biotechnol. Processes. 3: 315-343.

33. McDonald & Burke. (1984). J. Gen. Microbiol. 130: 203-208

## Claims

1. Genetically engineered Bacillus sphaericus (B. sph.) transformed with a DNA sequence from Bacillus thuringiensis var. israelensis (B.t.i.), encoding the B.t.i. insect larvicidal toxin proteins.

2. B. sph. according to Claim 1 expressing both the B. sph. 110kDa endotoxins and a B.t.i. insect larvicidal toxin protein.

3. B. sph. according to Claim 2 wherein said toxin protein is selected from the 28kDa, 65kDa, 128kDa and 135kDa toxin protein from B.t.i.

4. B. sph. according to Claim 1, 2 or 3 wherein said DNA sequence is obtained from the 72-75 megadalton, 112 kilobases plasmid.

5. B. sph. according to Claim 4 containing the Hind III, 9.7 kilobase DNA sequence of the B.t.i. toxin gene.

6. B. sph. according to Claim 4 wherein said DNA sequence is inserted in a plasmid derived from pPL603E.

7. B. sph. CNCM I-746.

8. B. sph. according to Claim 1 transformed with the plasmid which is contained in B. sph. CNCM I-746 which is a derivative of pPL603E which has the B.ti.i. toxin gene inserted therein.

9. A plasmid including a DNA sequence encoding the B.t.i. insect larvicidal toxin protein.

10. A plasmid according to Claim 9 derived from pPL603E.

11. A plasmid according to Claim 9 wherein said DNA sequence is a Hind III 9.7 kb segment.

12. An insect larvicidal composition comprising an effective amount of spores of B.t.i. toxin produced by genetically engineered B. sph. or spores of B. sph. transformed with a DNA sequence from B.t.i. encoding the B.ti.i. insect larvicidal toxin or a combination of the two, together with a carrier compatible with both the spores and the water environment to which it is to be applied.

13. A dry composition according to Claim 12.

14. A liquid composition according to Claim 12.

15. A method of combatting insects selected from mosquitos and black flies comprising applying to the aqueous medium in which the larvae of the insects live, an effective amount of a genetically engineered B. sph. of Claim 1.

16. A method according to Claim 1 comprising applying to the aqueous medium in which the larvae of the insects live, an effective amount of a composition according to Claim 12.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 153 166 (SYNTRO CORP.) * Claims * | 1-16 | C 12 N 15/00 A 01 N 63/02 C 12 N 1/20 // (C 12 P 1/04 C 12 R 1:07 ) |
| Y | EP-A-0 195 285 (UNIVERSITY OF GEORGIA RES.) * Claims * | 1-16 | |
| Y | BIOTECHNOLOGY AND GENETIC ENGINEERING REVIEWS, vol. 2, October 1984, pages 341-363, Intercept Ltd; D.H. DEAN: "Biochemical genetics of the bacterial insect-control agent bacillus thuringiensis: Basic principles and prospects for genetic engineering" * Page 357, lines 12-18 * | 1-16 | |
| D,Y | CHEMICAL ABSTRACTS, vol. 101, no. 7, 13th August 1984, page 133, abstract no. 49329g, Columbus, Ohio, US; K.O. McDONALD et al.: "Plasmid, transformation of Bacillus sphaericus 1593", & J. GEN. MICROBIOL. 1984, 130(1), 203-8 * Abstract * | 1-16 | TECHNICAL FIELDS SEARCHED (Int. Cl.4) C 12 N C 12 P C 12 R |
| D,A | J. MOL. BIOL., vol. 191, 1986, pages 13-22, Academic Press Inc. Ltd, London, GB; E.S. WARD et al.: "Bacillus thuringiensis var. israelensis delta-Endotoxin. Cloning and expression of the toxin in sporogenic and asporogenic strains of bacillus subtilis" | | |

The present search report has been drawn up for all claims

| Place of search | Date of completion of the search | Examiner |
|---|---|---|
| THE HAGUE | 20-07-1989 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document